(19) Europäisches Patentamt
European Patent Office
Office européen des brevets

(11) Veröffentlichungsnummer: **0 017 896**
**B1**

(12) **EUROPÄISCHE PATENTSCHRIFT**

(45) Veröffentlichungstag der Patentschrift: **15.12.82**

(51) Int. Cl.$^3$: **C 07 C131/08**

(21) Anmeldenummer: **80101860.7**

(22) Anmeldetag: **08.04.80**

(54) **1-Nitro-2-amino-3-oximino-1-cyclohexene und Verfahren zu ihrer Herstellung.**

(30) Priorität: **11.04.79 DE 2914672**

(43) Veröffentlichungstag der Anmeldung: **29.10.80 (Patentblatt 80/22)**

(45) Bekanntmachung des Hinweises auf die Patenterteilung: **15.12.82 Patentblatt 82/50**

(84) Benannte Vertragsstaaten: **BE CH DE FR GB LI**

(56) Entgegenhaltungen:
**HOUBEN-WEYL: « METHODEN DER ORGANISCHEN CHEMIE », 4e Auflage, Band XI/1, Stickstoffverbindungen II, Georg Thieme Verlag STUTTGART (DE), Seite 111 « Aminolyse der enolischen Hydroxylgruppe »**

(73) Patentinhaber: **BASF Aktiengesellschaft**
**Carl-Bosch-Strasse 38**
**D-6700 Ludwigshafen (DE)**

(72) Erfinder: **Fischer, Rolf, Dr.**
**Bergstrasse 98**
**D-6900 Heidelberg (DE)**
Erfinder: **Weitz, Hans-Martin, Dr.**
**Auf dem Koeppel 40**
**D-6702 Bad Duerkheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 017 896 B1

1-Nitro-2-amino-3-oximino-1-cyclohexane und Verfahren zu ihrer Herstellung

Die Erfindung betrifft die neuen 1-Nitro-2-amino-3-oximino-1-cyclohexene und ein Verfahren zu ihrer Herstellung durch Umsetzung von 1-Nitro-2-oxo-3-oximinocyclohexanen mit Ammoniumsalzen von Carbonsäuren.

Aus der US-Patentschrift 3 732 303 und der deutschen Offenlegungsschrift 1 926 857 ist bekannt, daß sich 1-Nitro-2-oxo-3-oximinocyclohexan mit wasserfreiem oder wäßrigem Ammoniak zu 2-Oximino-6-nitrocapronsäureamid spalten läßt.

Es wurde nun gefunden, daß man 1-Nitro-2-amino-3-oximino-1-cyclohexene der Formel

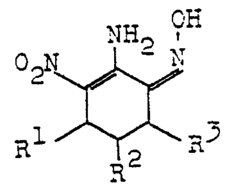

(I)

in der die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, vorteilhaft erhält, wenn man 1-Nitro-2-oxo-3-oximinocyclohexane der Formel

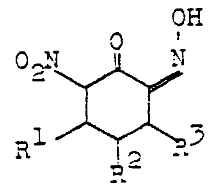

(II)

in der $R^1$, $R^2$ und $R^3$ die vorgenannte Bedeutung bezitzen, mit 1 bis 20 Äquivalenten Ammoniumsalz einer Carbonsäure je Mol Ausgangsstoff II umsetzt.

Weiterhin wurden die neuen 1-Nitro-2-amino-3-oximino-1-cyclohexene der Formel

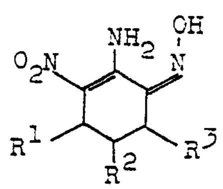

(I)

in der die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, gefunden.

Die Umsetzung wird für den Fall der Verwendung von 1-Nitro-2-oxo-3-oximinocyclohexan und Ammoniumacetat durch die folgenden Formeln wiedergegeben :

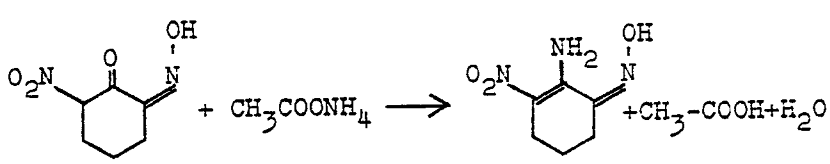


Das Verfahren nach der Erfindung liefert in einstufiger Reaktion auf einfachem und wirtschaftlichem Wege die bisher nicht beschriebenen 1-Nitro-2-amino-3-oximino-1-cyclohexene in guter Ausbeute und Reinheit.

Die als Ausgangsstoffe verwendeten 1-Nitro-2-oxo-3-oximinocyclohexane lassen sich beispielsweise nach den in der deutschen Offenlegungsschrift 2 153 764 und der US-Patentschrift 3 681 460 beschriebenen Verfahrenweisen herstellen. Der Ausgangsstoff II wird mit 1 bis 20 Äquivalenten, vorzugsweise 1 bis 10 Äquivalenten Ammoniumsalz einer Carbonsäure je Mol Ausgangsstoff II, umgesetzt.

Bevorzugte Ausgangsstoffe II und dementsprechend bevorzugte Endstoffe 1 sind solche, in deren Formeln $R^1$, $R^2$ und $R^3$ gleich oder verschieden sind und jeweils ein Wasserstoffatom, einen Alkylrest mit 1 bis 8 Kohlenstoffatomen, einen Cycloalkylrest mit 5 bis 8 Kohlenstoffatomen, einen Aralkylrest oder

Alkylarylrest mit 7 bis 12 Kohlenstoffatomen oder einen Phenylrest bedeuten. Die genannten Reste können noch durch unter den Reacktionsbedingungen inerte Gruppen, z.B. Alkylgruppen oder Alkoxygruppen mit jeweils 1 bis 4 Kohlenstoffatomen, substituiert sein.

Beispielsweise sind folgende 1-Nitro-2-oxo-3-oximinocyclohexane als Ausgangsstoffe II geeignet : 1-Nitro-2-oxo-3-oximinocyclohexan ; in 4-, 5- oder 6-Stellung einfach oder in 4, 5-, 4, 6- oder 5, 6-Stellung gleich oder unterschiedlich zweifach oder in 5, 6-Stellung gleich oder unterschiedlich dreifach durch die Methyl-, Äthyl-, Propyl-, Isopropyl-, Butyl-, Isobutyl-, sek.-Butyl-, tert.-Butyl-, Pentyl-, Hexyl-, Heptyl-, Octyl-, Cyclopentyl-, Cyclohexyl-, Benzyl-, Phenyl-, o-Methylphenyl-, m-Methylphenyl-, p-Methylphenyl, o-Methoxyphenyl-, m-Methoxyphenyl-, p-Methoxyphenylgruppe substituiertes 1-Nitro-2-oxo-3-oximinocyclohexan.

Die Umsetzung wird im allgemeinen bei einer Temperatur von − 20 bis + 200 °C, vorzugsweise von + 20 bis 150 °C, drucklos oder unter Druck, diskontinuierlich oder kontinuierlich durchgeführt. Im allgemeinen verwendet man unter den Reaktionsbedingungen inerte, zweckmäßig organische Lösungsmittel, vorzugsweise Carbonsäuren. Bevorzugt benutzt man die Carbonsäure als Lösungsmittel, die auch dem verwendeten Ammoniumsalz zugrundeliegt. Zweckmäßig verwendet man das Lösungsmittel in einer Menge von 5 bis 70, vorteilhaft von 10 bis 50 Äquivalenten, vorzugsweise 20 bis 40 Äquivalenten, bezogen auf 1 Mol Ausgangsstoff II.

Die den Ammoniumsalzen zugrundeliegenden Carbonsäuren können einbasische Säuren oder auch äquivalente Mengen mehrbasischer Säuren sein. Beispielsweise sind folgende Säuren geeignet : aliphatische Carbonsäuren wie Chloressigsäure, Dichloressigsäure, Trichloressigsäure, Oxalsäure, Ameisensäure, Essigsäure, Propionsäure, Buttersäure, Isobuttersäure, Glykolsäure, Milchsäure, Weinsäure, Zitronensäure, Caprylsäure, Trimethylessigsäure, β-Chlorpropionsäure, Bernsteinsäure, Malonsäure, Isovaleriansäure, Valeriansäure, Glutarsäure, Adipinsäure ; cycloaliphatische, araliphatische, aromatische Carbonsäuren wie Benzoesäure, Phenylpropionsäure, Cyclohexancarbonsäure, Phenylessigsäure, Phthalsäure, p.-Toluylsäure, p-Nitrobenzoesäure. Bevorzugt sind die Ammoniumsalze aliphatischer Carbonsäuren mit 1 bis 18, insbesondere 1 bis 8 Kohlenstoffatomen.

Die Reaktion kann wie folgt durchgeführt werden : Ein Gemisch aus Ausgangsstoff II, Ammoniumcarboxylat und Lösungsmittel wird 1 bis 20 Stunden bei der Reaktionstemperatur gehalten. Der Endstoff wird dann in üblicher Weise aus dem Reaktionsgemisch isoliert, z.B wird das Lösungsmittel anschließend im Vakuum abdestilliert, das zurückbleibende Reaktionsgemisch mit Wasser digeriert und der gebildete kristalline Niederschlag abgesaugt.

Die nach dem Verfahren der Erfindung herstellbaren neuen Endstoffe I sind wertvolle Ausgangsstoffe für die Herstellung von Farbstoffen, Pflanzenschutzmitteln, Pharmazeutika, Komplexbildnern für Schwermetallionen, von Triaminen und stickstoffhaltigen Heterocyclen. 1-Nitro-2-amino-3-oximino-1-cyclohexen läßt sich durch Erhitzen mit Wasser in Gegenwart von Ammoniak selektiver als nach den zum Stand der Technik genannten Verfahrensweisen zu 2-Oximino-6-nitrocapronsäureamid, einem Vorprodukt der Aminosäure Lysin, umsetzen.

Die in den Beispielen angeführten Teile bedeuten Gewichtsteile.

Beispiel 1

Ein Gemisch aus 17,2 Teilen 1-Nitro-2-oxo-3-oximinocyclohexan, 77 Teilen Ammoniumacetat und 180 Teilen Eisessig wird unter Rühren 14 Stunden lang auf 85 °C erhitzt. Dann zieht man den Eisessig bei einer Badtemperatur von 60 bis 65 °C und 2 mbar ab, digeriert den Rückstand 2 Stunden lang mit 500 Teilen Eiswasser und saugt den Kristallbrei auf einer Glasfilternutsche ab. Man erhält 12,4 Teile 1-Nitro-2-amino-3-oximino-1-cyclohexen (72 % der Theorie) vom Schmelzpunkt 207 °C (aus Wasser).

Beispiel 2

Wie in Beispiel 1 beschrieben, werden 17,2 Teile 1-Nitro-2-oxo-3-oximinocyclohexan mit 75 Teilen Ammoniumformiat in 200 Teilen Ameisensäure bei Rückflußtemperatur (100 °C) umgesetzt. Man erhält 11,7 Teile 1-Nitro-2-amino-3-oximino-1-cyclohexen (68 % der Theorie) vom Schmelzpunkt 205 bis 206 °C (aus Wasser).

**Ansprüche**

1. Verfahren zur Herstellung von 1-Nitro-2-amino-3-oximino-1-cyclohexenen der Formel

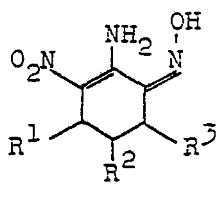

(I)

in der die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, dadurch gekennzeichnet, daß man 1-Nitro-2-oxo-3-oximinocyclohexane der Formel

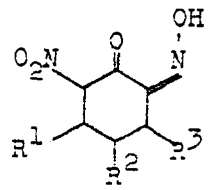

(II)

in der $R^1$, $R^2$ und $R^3$ die vorgenannte Bedeutung besitzen, mit 1 bis 20 Äquivalenten Ammoniumsalz einer Carbonsäure je Mol Ausgangsstoff II umsetzt.

2. 1-Nitro-2-amino-3-oximino-1-cyclohexen der Formel

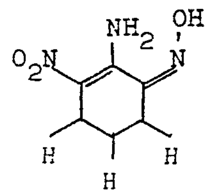

(I)

**Claims**

1. A process for the production of 1-nitro-2-amino-3-oximino-1-cyclohexenes of the formula

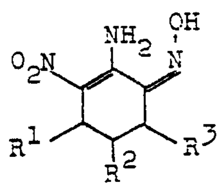

(I)

where the individual radicals $R^1$, $R^2$ and $R^3$ may be identical of different and each is hydrogen or an aliphatic, cycloaliphatic, araliphatic or aromatic radical, wherein a 1-nitro-2-oxo-3-oximinocyclohexane of the formula

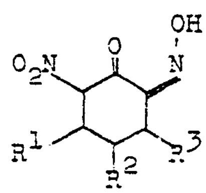

(II)

where $R^1$, $R^2$ and $R^3$ have the above meanings, is reacted with 1 to 20 equivalents of ammonium salt of a carboxylic acid per mole of starting material II.

2. 1-Nitro-2-amino-3-oximino-1-cyclohexene of the formula

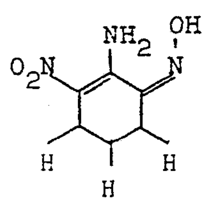

(I)

**Revendications**

1. Procédé pour la préparation de 1-nitro-2-amino-3-oximino-1-cyclohexènes de formule

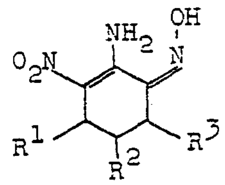

(I)

dans laquelle les différents radicaux $R^1$, $R^2$ et $R^3$ peuvent être semblables ou différents et représentent chacun un atome d'hydrogène, un radical aliphatique, cycloaliphatique, araliphatique ou aromatique, caractérisé en ce qu'on fait réagir des 1-nitro-2-oxo-3-oximinocyclohexanes de formule

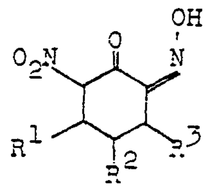

(II)

dans laquelle $R^1$, $R^2$ et $R^3$ ont les significations données ci-dessus, avec 1 à 20 équivalents d'un sel ammonique d'un acide carboxylique par mole de substance de départ II.

2. 1-nitro-2-amino-3-oximino-1-cyclohexène de formule

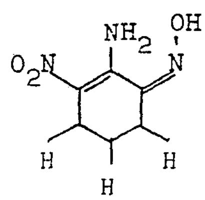

(I)